# EUROPEAN PATENT SPECIFICATION

(11) **EP 1 423 071 B1**
(45) Date of publication and mention of the grant of the patent: **16.11.2005**
(21) Application number: 02768245.9
(22) Date of filing: 22.08.2002
(51) Int. Cl.: A61F 13/15

(54) **ABSORBENT ARTICLE HAVING OPENINGS IN THE ABSORBENT BODY**
SAUGFÄHIGER ARTIKEL MIT ÖFFNUNGEN IM SAUGFÄHIGEN KÖRPER
ARTICLE ABSORBANT AVEC ELEMENT ABSORBANT COMPORTANT DES OUVERTURES

(30) Priority: 31.08.2001 SE 0102897
(43) Date of publication of application: 02.06.2004
(73) Proprietor: SCA Hygiene Products AB, 405 03 Göteborg (SE)
(72) Inventor: VARTIAINEN, Kent, S-443 38 Lerum (SE)
(74) Representative: Romare, Laila Anette
(86) International application number: PCT/SE2002/001494
(87) International publication number: WO 2003/017900

(56) References cited:
- SE-B- 345 378
- SE-B- 432 348
- US-A- 3 814 101
- US-A- 5 533 991
- US-A- 5 961 505

## Description

### TECHNICAL FIELD

The present invention relates to an absorbent article, such as a nappy or an incontinence pad, comprising a liquid-permeable surface layer, a liquidtight surface layer and a first absorbent body arranged between the surface layers. The first absorbent body extends in a longitudinal direction and a transverse direction along the surface layers and also a through-direction from the liquid-permeable surface layer towards the liquidtight surface layer the first absorbent body having openings which extend through the first absorbent body in the through-direction, and a liquid-penetrable layer being arranged on that side of the first absorbent body which faces away from the liquid-permeable surface layer.

### BACKGROUND ART

US 5,846,231 describes an absorbent article comprising a liquid-permeable surface layer attached to the liquidtight surface layer through openings in the form of cuts in the absorbent body. In order to obtain better spreading across the article, the cuts are arranged intermittently in a longitudinal direction also in addition to, as previously known, in a transverse direction. For effective spreading and handling of large quantities of urine and also more viscous liquids such as loose motions, however, this solution is inadequate.

WO 99/25291 describes an absorbent article comprising a receiving means intended to be positioned close to the anus and having a stated effective openness and also arranged over a storage layer for dealing with more viscous liquids such as loose motions. The liquid-permeable surface layer can be provided with holes. However, this solution is not adequate for effectively spreading and storing both urine and motions either.

US 3,814,101 discloses a disposable absorbent article having a perforated (slitted) top-sheet which is bonded to an underlying absorbent layer. The bonds are not synchronised with the slits in the top-sheet, but some of the bonds randomly overlap with some of the slits causing the bonds to extend somewhat down into the absorbent layer.

WO 96/20670 describes an absorbent article comprising an absorbent body having receiving spaces which may be in the form of holes through the absorbent body. Areas in the absorbent body adjacent to the receiving spaces consist of a material which, when wetted, increases in thickness in a direction through the article at right angles to the receiving surface of the article. The spaces are arranged between an overlying layer and an underlying layer.

### OBJECT AND MAJOR CHARACTERISTICS OF THE INVENTION

One object of the present invention is to provide an absorbent article with an improved capacity to deal with both urine and more viscous liquids such as loose motions. Another object of the present invention is to produce a drier contact surface against the skin when urine and more viscous liquids are dealt with.

In accordance with the invention, an absorbent article of the type referred to in the introduction has been produced. An absorbent article according to the invention is characterized in that the liquid-permeable surface layer is attached to the liquid-permeable layer in the openings through the first absorbent layer.

Attached to means that the materials are joined together with one another directly, or indirectly via an intermediate material. The attachment can be in the form of, for example, ultrasonic welding, thermal welding or glue.

The liquid-permeable surface layer can consist of any known material intended for the purpose of allowing liquid to pass through to an underlying layer. Examples of such materials are non-woven material, perforated plastic film, perforated hydrophobic materials, net or the like. The surface layer can also consist of a laminate of two or more layers of the same material or different materials.

According to an embodiment of the invention, the liquid-permeable surface layer is not attached to the first absorbent body. The liquid-permeable surface layer in modem known absorbent articles is usually attached to an underlying absorbent structure by means of glue. This is done inter alia so that the absorbent structure will not move or form lumps. By virtue of attaching the liquid-permeable surface layer to the second absorbent body, it is not necessary to glue the liquid-permeable surface layer to the first absorbent structure. Problems such as glue breakthrough, owing to the application of too much glue to the article during manufacture, and attendant skin irritations or restricted liquid permeability as pores in the liquid-permeable surface layer are obstructed by glue, can thus be eliminated. Other problems such as stoppages during manufacture of the article on account of glue in the machines, and impaired absorption capacity as a result of the glue having blocked pores in the absorbent body can also be reduced. Advantageously, it is possible to use thin liquid-permeable surface layers without the pores of the surface layer being clogged or glue penetrating the material, as a result of which better permeability can be obtained. The liquid-permeable surface layer can thus have a maximum weight per unit area of 20 g/m².

According to an embodiment, a liquid transport layer is arranged between the liquid-permeable surface layer and the first absorbent body. In this embodiment, the liquid transport layer is also attached to the second absorbent structure through the openings through the first absorbent body. The liquid-permeable surface layer is attached indirectly to the second absorbent structure through the liquid transport layer lying between the surface layer and the second absorbent structure.

According to an embodiment, the second absorbent structure comprises a liquid-penetrable layer arranged on the liquid-receiving side of the structure, to which layer the liquid-permeable surface layer is attached. The liquid-permeable layer can consist of any known material intended for the purpose of allowing liquid to pass through to an adjacent layer. Examples of such materials are non-woven material, perforated plastic film, perforated hydrophobic materials, net or the like. The layer can also consist of a laminate of two or more layers of the same material or different materials. As this layer is not in direct contact with skin, it can also advantageously consist of a hydrophilic material or a hydrophilicized hydrophobic material and thus rapidly allow liquid to pass through to underlying layers. Examples of suitable hydrophilic materials are rayon or cotton. Examples of methods of hydrophilicizing hydrophobic materials are to treat the material with wetting agent, flame-treatment, corona or plasma. The second absorbent structure can consist of a liquid-penetrable layer.

The liquid-penetrable layer does not have to be attached to underlying layers such as, for example, any other parts of the absorbent structure, or to an underlying third absorbent structure or alternatively to the liquidtight surface layer in embodiments in which the second absorbent structure consists of only a liquid-penetrable layer. By virtue of the fact that the liquid-penetrable layer is not attached to the underlying layer, a flexible space is obtained between the liquid-penetrable layer and the underlying layer. The liquid-permeable surface layer and the liquid-penetrable layer can then preferably have holes which are arranged at the openings through the absorbent body. Motions and urine can thus collect in the flexible space between the liquid-penetrable layer and the underlying layer after passing through the holes and openings.

In the above paragraph, underlying layer means that the layer is arranged on a side of the liquid-penetrable layer which faces away from a wearer during use.

In this context, not attached to means that the materials have not intentionally been joined together with one another during manufacture. However, the first absorbent body can, in an area closest to the attachment of the liquid-permeable surface layer to the second absorbent structure, be joined to either the liquid-permeable surface layer or the second absorbent structure or both. The materials can be joined together with one another along transverse and/or longitudinal side edges.

The liquid-permeable surface layer can have hydrophobic areas between the openings. Urine and motions then run down rapidly into the openings for collection before further spreading into absorbent structures and bodies. The liquid-permeable surface layer can thus provide a drier feeling during use. The liquid-permeable surface layer can consist of a perforated hydrophobic material, or the surface layer can be treated chemically so as to obtain a zoned hydrophobic surface in a manner which is previously known.

### BRIEF DESCRIPTION OF FIGURES

The invention will be described in greater detail below with reference to figures, where
- Figure 1: shows a plane view of a nappy according to an embodiment of the invention,
- Figure 2a: shows a section through the nappy in Figure 1 along the line II-II according to a first embodiment,
- Figure 2b: shows a section through the nappy in Figure 1 along the line II-II according to a second embodiment,
- Figure 2c: shows a section through the nappy in Figure 1 along the line II-II according to a third embodiment,
- Figure 2d: shows a section through the nappy in Figure 1 along the line II-II according to a fourth embodiment,
- Figure 2e: shows a section through the nappy in Figure 1 along the line II-II according to a fifth embodiment, and
- Figure 2f: shows a section through the nappy in Figure 1 along the line II-II according to a sixth embodiment

### MODES FOR CARRYING OUT THE INVENTION

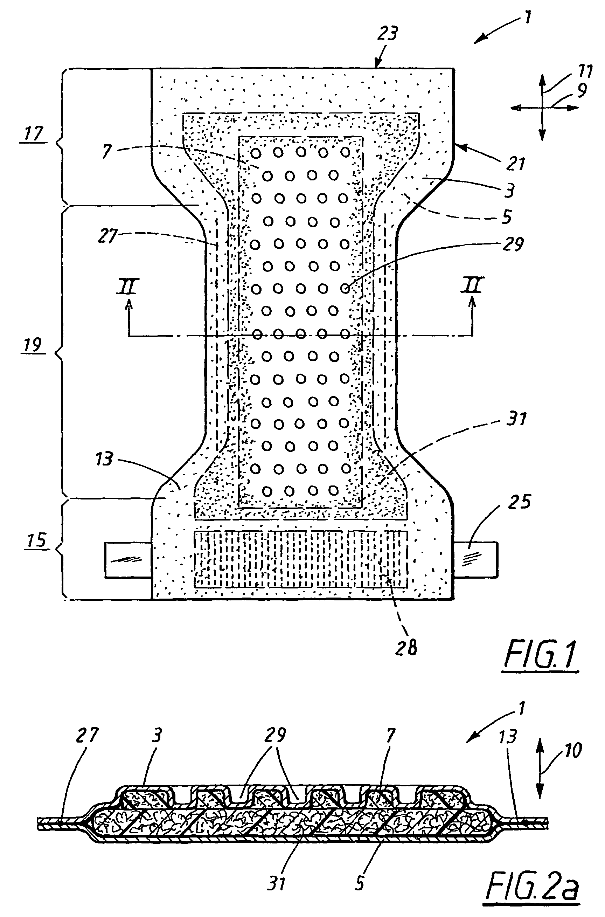

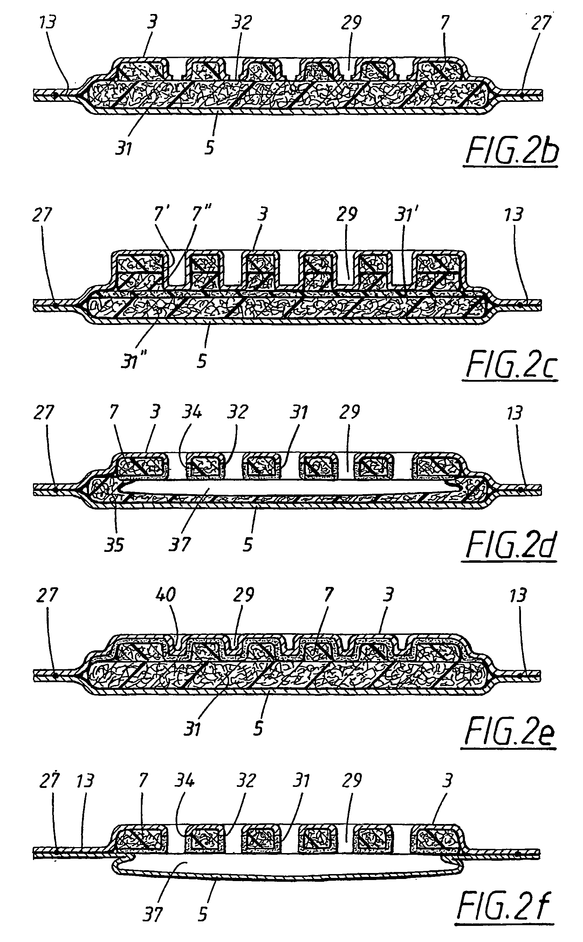

The nappy 1 shown in Figures 1 and 2 comprises a liquid-permeable surface layer 3, a liquidtight surface layer 5 and also a first absorbent body 7 arranged between the surface layers 3, 5. The nappy has a transverse direction 9 and a longitudinal direction 11 in a plane. The nappy also has a through-direction 10 at right angles to the longitudinal and the transverse direction.

The two surface layers extend outside the first absorbent body 7 in the transverse direction 9 and the longitudinal direction 11 and are interconnected in the projecting portions 13 by, for example, glue or thermal or, alternatively, ultrasonic welding.

The liquid-permeable surface layer 3 can consist of any known material intended for the purpose of allowing liquid to pass through to an underlying layer. Examples of such materials are non-woven material, perforated plastic film, perforated hydrophobic materials, net or the like. The surface layer can also consist of a laminate of two or more layers of the same material or different materials. The liquid-permeable surface layer can consist of a stretchable material, either elastically stretchable or inelastically stretchable.

The liquidtight surface layer 5 can consist of any material intended for the purpose of resisting liquid penetration. Examples of such materials are plastic films, laminates of a number of layers of non-woven material and/or plastic film and similar materials. It is an advantage if the material has the capacity to allow water vapour to pass through and thus exhibits breathability.

The nappy 1 has an elongate shape with a rear portion 15 intended to lie close to a wearer at the rear during use, a front portion 17 intended to lie close to the wearer at the front during use and a crotch portion 19 lying between the front portion 17 and the rear portion 15. The crotch portion 19 lying between the front portion 17 and the rear portion 15 is narrower than the rear portion 15 and the front portion 17, the nappy 1 having an hourglass shape. The nappy also has longitudinal side edges 21 and transverse end edges 23.

On the rear portion 15, the nappy has an attachment means 25 arranged along each longitudinal side edge 21 and intended to attach to a receiving area arranged on a side of the front portion 17 which faces outwards during use of the nappy. Such attachment means 25 are previously known and can comprise, for example, adhesive fastening means such as, for example, tape or mechanical fastening means such as, for example, touch and close fasteners. The receiving area can, for adhesive fastening means, be reinforced with a plastic film or, for mechanical fastening means, constitute a surface to which mechanical fastening means can attach, such as, for example, touch and close fasteners or non-woven material.

The design of nappy described above and shown in the drawings is not to be considered as limiting the invention. Within the scope of the invention, it is not necessary, for example, for the absorbent article to be provided with attachment means. An article having closed side portions such as, for example, nappy pants is also a possible embodiment of the invention. Absorbent inserts for application in liquidtight coverings are also possible embodiments of the invention. The design is of course not to be considered as being limited to the hourglass shape shown either.

The nappy 1 also has longitudinal elastic means 27 attached in a pretensioned state along each longitudinal side edge 21. These elastic means 27 are intended to seal against the legs of a wearer during use and thus provide better protection against leakage of liquids from the nappy. Along the end edge 23 on the rear portion 15, the nappy 1 has a transverse elastic means 28 attached to the nappy in a pretensioned state. This transverse elastic means 28 constitutes the waist elastic. It is not necessary for the invention that the article has elastic means.

The first absorbent body can comprise one or more layers of the same absorbent material or different absorbent materials.

The first absorbent body is preferably designed so as to receive and transport liquid. It can then comprise a layer of a material which has large pores or capillaries so as to acquire a low resistance to liquid flow through the body. This layer suitably consists of a soft material which feels pleasant against the body of the wearer during use. Examples of materials which can be used are cellulose layers with a low degree of compression, in particular made of mechanical, thermomechanical or chemithermomechanical pulp (CTMP) or fibre mats and wadding made of other types of natural or synthetic fibres. It is also possible to use soft perforated or open-cell materials such as, for example, foam.

The first absorbent body can also comprise a certain quantity of highly absorbent polymers, what are known as superabsorbents, in the form of fibres, particles, granules, film or the like. These superabsorbents are characterized by a great capacity to bind liquid in a quantity corresponding to several times their own weight.

The first absorbent body can also comprise a material which expands greatly in the through-direction when wetted. The production of a suitable such material is described in WO 94/10956. The characteristic feature of this material is that it is produced by dry-forming flash-dried cellulose fibres to form a web with a weight per unit area of 30-2000 g/m² which is compressed to a density between 0.2 and 1 g/m³, and that the web is incorporated as an absorbent structure into an absorbent article without subsequent defibration and fluff formation. Another example is cellulose fluff pulp, into which a certain quantity of superabsorbent material, preferably at least. 10% by weight, has been mixed. Examples of other materials which can expand in the through-direction are compressed foamed materials and fibre wadding which partly return to their uncompressed size when wetted. The liquid-permeable surface layer preferably consists of a stretchable material as the first absorbent body comprises an expandable material.

The first absorbent body 7 has a number of openings 29 in the form of rounded holes. These openings extend through the first absorbent body 7 in the through-direction 10 from that side of the absorbent body which faces the wearer during use of the article to that side of the absorbent body which faces away from the wearer during use of the article. The shape of the openings is not critical, but the openings should be sufficiently large for collecting motions. The openings 29 can also be in the form of cuts through the absorbent body.

The openings 29 can be arranged over essentially the whole of the first absorbent body 7, as in Figure 1. However, this is not necessary for the invention. The openings can instead be arranged, for example, within one or more areas of the absorbent body, for example within the front portion, the rear portion, the crotch portion or parts of these. Nor do the openings have to be the same size. For example, the openings in the front portion and the rear portion can be larger than the openings in the crotch portion, or vice versa. The openings in the rear portion can be larger than the openings in the front portion, or vice versa. The openings within an area can also be of different size. The number of openings is preferably at least three, preferably more, but only one or two opening(s) are also to be seen as possible embodiments of the invention.

In accordance with the invention, the nappy 1 comprises a second absorbent structure 31 arranged between the first absorbent body 7 and the liquidtight surface layer 5.

The liquid-permeable surface layer 3 is attached to the second absorbent structure 31 by means of, for example, ultrasound in areas which are constituted by the through-openings 29 in the first absorbent body 7. Thermal welding or gluing are examples of other possible ways of attaching the liquid-permeable surface layer to the second absorbent body 31.

It is not necessary for the first absorbent body to be attached to the second absorbent structure in any other way than by virtue of the liquid-permeable surface layer being attached to the second absorbent structure in the openings through the first absorbent body, as a result of which the first absorbent body is held in place between the liquid-permeable surface layer and the second absorbent structure. The openings then constitute a flexible space for collecting motions as they have the possibility to expand in the transverse, longitudinal and/or through-direction of the nappy because the first absorbent body is not attached to adjacent layers between the openings, as a result of which its structure acquires a certain flexibility/mobility.

The second absorbent structure can comprise a liquid-penetrable layer and/or one or more layers of the same absorbent material or different absorbent materials.

The second absorbent structure can advantageously function as a spreading layer and storage layer and suitably comprises a material of great density and with a great liquid-spreading and liquid-retaining capacity. An example of a suitable material is chemically produced cellulose fluff pulp (CP) with a weight per unit area of roughly 400 g/m². The dry-formed fibrous layer mentioned above and described in WO 94/10956 can also advantageously be used. The liquid-spreading layer can be provided with compression patterns so as to guide the liquid transport along the compressions.

The second absorbent structure 31 can comprise a material with an open structure which has large pores or capillaries so as to acquire a low resistance to liquid flow through the body. Examples of materials which can be used are cellulose layers with a low degree of compression or fibre mats and wadding made of other types of natural or synthetic fibres. It is also possible to use soft perforated or open-cell materials. Folded materials, undulating materials or materials shaped into an open structure in another way can also be used.

The second absorbent structure can also advantageously comprise some form of superabsorbent. A suitable content of superabsorbent can be at least 10% by weight, preferably 10-60% by weight.

The second absorbent structure can comprise a liquid-penetrable layer. This can consist of any known material suitable for the purpose. Examples of such materials are non-woven materials, perforated plastic films, net etc. The layer can be made of a hydrophilic material such as, for example, corona-treated non-woven or rayon.

The embodiment shown in Figure 2a of a cross section of the nappy in Figure 1 along the line II-II comprises a first absorbent body 7 comprising a layer which has large pores or capillaries so as to acquire a low resistance to liquid flow through the body. This layer suitably consists of a soft material which feels pleasant against the body of the wearer during use. Examples of materials which can be used are cellulose layers with a low degree of compression, in particular made of mechanical, thermomechanical or chemithermomechanical pulp (CTMP) or fibre mats and wadding made of other types of natural or synthetic fibres. It is also possible to use soft perforated or open-cell foamed materials. A liquid-permeable surface layer 3, which during use is arranged on that side of the first absorbent body 7 which faces a wearer of the nappy during use, is attached to a second absorbent structure 31 positioned on that side of the first absorbent body 7 which faces away from the wearer during use through openings 29 through the first absorbent body. These openings 29 extend through the first absorbent body 7 in a through-direction 10 from that side of the first absorbent body 7 which faces the wearer during use of the article to that side of the absorbent body which faces away from the wearer during use of the article.

The second absorbent structure 31 comprises, for example, a spreading layer/storage layer made of a material of great density and with a great liquid-spreading and liquid-retaining capacity. An example of a suitable material is chemically produced cellulose fluff pulp (CP) with a weight per unit area of roughly 400 g/m². The dry-formed fibrous layer mentioned above and described in WO 94/10956 can also advantageously be used. The second absorbent structure preferably also comprises 10-60% by weight superabsorbents.

The liquid-permeable surface layer in the embodiment shown in Figure 2b of a cross section of the nappy in Figure 1 along the line II-II has holes 32 at the openings 29 through the first absorbent body 7. The holes in Figure 2b are arranged straight over the openings through the first absorbent body. These holes can be formed in conjunction with the joining together of the liquid-permeable surface layer and the second absorbent structure, for example during welding, or a previously perforated liquid-permeable surface layer can be used. It is not necessary for the holes through the liquid-permeable surface layer to be arranged straight above the openings through the first absorbent body, but the holes should be positioned at the openings in such a manner as makes it possible for urine and/or motions to pass through the holes after collecting in the openings. The holes can be positioned, for example, so that they are directed towards the first absorbent body.

The holes 32 and the openings 29 are preferably sufficiently large for motions to be capable of passing through.

The second absorbent structure 31 in Figure 2b suitably comprises, on that side of the structure which faces the first absorbent body, a layer of a material which has large pores or capillaries so as to acquire a low resistance to liquid flow through the body. The layer preferably has an open structure suitable for collecting motions. Examples of materials which can be used are cellulose layers with a low degree of compression or fibre mats and wadding made of other types of natural or synthetic fibres. It is also possible to use soft perforated or open-cell materials. Folded materials, undulating materials or materials shaped into an open structure in another way can also be used.

The first absorbent body 7 in the embodiment shown in Figure 2c of a cross section of the nappy in Figure 1 along the line II-II comprises a first layer 7' of a material which has large pores or capillaries so as to acquire a low resistance to liquid flow through the body. This first layer 7' suitably consists of a soft material which feels pleasant against the body of the wearer during use. This first layer 7' is positioned against the liquid-permeable surface layer 3. The first absorbent body 7 also comprises a second absorbent layer 7" which is more open or porous than the first layer 7' and consists of a material which has large pores or capillaries so as to acquire a low resistance to liquid flow through the layer. Examples of materials which can be used are cellulose layers with a low degree of compression or fibre mats and wadding made of other types of natural or synthetic fibres. It is also possible to use soft perforated or open-cell materials. Folded materials, undulating materials or materials shaped into an open structure in another way can also be used.

A liquid-permeable surface layer 3, which during use is positioned on that side of the first absorbent body 7 which faces a wearer of the nappy during use, is attached to a second absorbent structure 31 positioned on that side of the first absorbent body 7 which faces away from the wearer during use through openings 29 through the first absorbent body. These openings 29 extend through the first absorbent body 7 in a through-direction 10 from that side of the first absorbent body 7 which faces the wearer during use of the article to that side of the absorbent body which faces away from the wearer during use of the article.

The second absorbent structure 31 in Figure 2c consists of, for example, a first layer 31' of hydrophilic non-woven material with a good admission capacity facing the first absorbent body 7 and a second layer 31" of a material of great density and with a great liquid-spreading and liquid-retaining capacity facing away from the first absorbent body. An example of a suitable material is chemically produced cellulose fluff pulp (CP) with a weight per unit area of roughly 400 g/m². The dry-formed fibrous layer mentioned above and described in WO 94/10956 can also advantageously be used. The absorbent structure can preferably comprise 10-60% by weight superabsorbents.

In Figure 2d, a liquid-permeable surface layer 3 is attached to a second absorbent structure 31 in the form of a liquid-penetrable layer positioned on that side of the first absorbent body 7 which faces away from the wearer during use through openings 29 through the absorbent body. These openings 29 extend through the first absorbent body 7 in a through-direction 10 from that side of the first absorbent body 7 which faces the wearer during use of the article to that side of the absorbent body which faces away from the wearer during use of the article. At holes 32 through the liquid-permeable surface layer, the surface layer is attached to the liquid-penetrable layer at holes 34 through the layer, which holes 32, 34 are positioned at the openings 29 through the first absorbent body so that urine and/or motions can pass through the openings 29 and the holes 32, 34.

A third absorbent structure 35 is positioned on that side of the second absorbent structure which faces away from the wearer during use. The third absorbent structure 35 in Figure 2d comprises, for example, a layer of a material which has large pores or capillaries so as to acquire a low resistance to liquid flow through the body. The layer preferably has an open structure suitable for collecting motions. Examples of materials which can be used are cellulose layers with a low degree of compression or fibre mats or wadding made of other types of natural or synthetic fibres. It is also possible to use soft perforated or open-cell materials. Folded materials, undulating materials or materials shaped into an open structure in another way can also be used.

The third absorbent structure 35 can comprise a material of great density and with a great liquid-spreading and liquid-retaining capacity. An example of a suitable material is chemically produced cellulose fluff pulp (CP) with a weight per unit area of roughly 400 g/m². The dry-formed fibrous layer mentioned above and described in WO 94/10956 can also advantageously be used. The third absorbent structure can also comprise superabsorbents, preferably in a quantity of 10-60% by weight.

The second absorbent structure 31 in Figure 2d is not attached to the third absorbent structure 35, a space 37 being formed between the second absorbent structure and the third absorbent structure for collecting motions after the motions have passed through the openings through the layers and the first absorbent body.

The embodiment shown in Figure 2e of a cross section of the nappy in Figure 1 along the line II-II comprises a liquid transport layer 40 arranged between the liquid-permeable surface layer 3 and the first absorbent body 7. In Figure 2e, both the liquid-permeable surface layer 3 and the liquid transport layer 40 are attached to the second absorbent structure 31 through openings 29 which extend in a through-direction through the first absorbent body 7. The liquid-permeable surface layer 3 is indirectly attached to the second absorbent structure by the liquid transport layer 40 lying between the liquid-permeable surface layer 3 and the second absorbent structure 31.

The liquid transport layer 40 preferably consists of a material which has large pores or capillaries so as to acquire a low resistance to liquid flow through the body. The liquid transport layer suitably consists of a soft material which feels pleasant against the body of the wearer during use.

Examples of materials which can be used are cellulose layers with a low degree of compression or fibre mats and wadding made of other types of natural or synthetic fibres. It is also possible to use soft perforated or open-cell materials, for example foam.

In Figure 2f, a liquid-permeable surface layer 3 is attached to a second absorbent structure 31 in the form of a liquid-penetrable layer positioned on that side of the first absorbent body 7 which faces away from the wearer during use through openings 29 through the absorbent body. These openings 29 extend through the first absorbent body 7 in a through-direction 10 from that side of the first absorbent body 7 which faces the wearer during use of the article to that side of the absorbent body which faces away from the wearer during use of the article. At holes 32 through the liquid-permeable surface layer, the surface layer is attached to the liquid-penetrable layer at holes 34 through the layer, which holes 32, 34 are positioned at the openings 29 through the first absorbent body so that urine and/or motions can pass through the openings 29 and the holes 32, 34.

A liquidtight surface layer 5 is arranged on that side of the liquid-penetrable layer which faces away from the wearer during use. The second absorbent structure 31 in Figure 2f is not attached to the liquidtight surface layer 5, a space 37 being formed between the second absorbent structure and the liquidtight surface layer 5 for collecting motions and urine after passing through the openings through the layers and the first absorbent body. Liquid can then also be absorbed into the first absorbent body from that side thereof which faces the liquidtight surface layer through the liquid-penetrable layer.

The invention is not to be considered as being limited to the embodiments indicated above, but a large number of modifications are possible within the scope of the following patent claims.

## Claims

1. Absorbent article, such as a nappy or an incontinence pad, comprising a liquid-permeable surface layer (3), a liquidtight surface layer (5), and a first absorbent body (7) which is arranged between the surface layers and extends in a longitudinal direction (11) and a transverse direction (9) along the surface layers and also a through-direction (10) from the liquid-permeable surface layer (3) towards the liquidtight surface layer (5), said first absorbent body (7) having openings (29) which extend through the first absorbent body (7) in the through-direction (10), and a liquid-penetrable layer (31) being arranged on that side of the first absorbent body (7) which faces away from the liquid-permeable surface layer (3), **characterized in that** the liquid-permeable surface layer (3) is attached to the liquid-penetrable layer (31) in the openings (29) through the first absorbent layer (7).

2. Absorbent article according to Claim 1, **characterized in that** the liquid-permeable surface layer (3) is not attached by being intentionally joined to the first absorbent body (7).

3. Absorbent article according to any one of the preceding claims, **characterized in that** the first absorbent body (7) is not attached by being intentionally joined to the liquid-penetrable layer (31).

4. Absorbent article according to any one of the preceding claims, **characterized in that** the openings (29) are in the form of rounded holes.

5. Absorbent article according to any one of the preceding claims, **characterized in that** the openings (29) are in the form of longitudinal channels.

6. Absorbent article according to any one of the preceding claims, **characterized in that** the liquid-permeable surface layer (3) has holes (32) arranged at the openings (29) through the first absorbent body so that urine and/or motions can pass through the holes (32) and the openings (29).

7. Absorbent article according to any one of the preceding claims, **characterized in that** the liquid-permeable surface layer (3) comprises hydrophobic areas between the openings (29) through the first absorbent body (7).

8. Absorbent article according to any one of the preceding claims, **characterized in that** the liquid-permeable surface layer (3) has a maximum weight per unit area of 20 g/m².

9. Absorbent article according to any one of the preceding claims, **characterized in that** a liquid transport layer (40) is arranged between the liquid-permeable surface layer (3) and the first absorbent body (7), the liquid transport layer (40) also being attached to the liquid-penetrable layer (31) through the openings (29).

10. Absorbent article according to any one of the preceding claims, **characterized in that** a flexible space (37) is formed between the liquid-penetrable layer (31) and an underlying layer (5).

## Patentansprüche

1. Absorbierender Gegenstand, wie beispielsweise eine Windel oder eine Inkontinenzschutz, umfassend eine flüssigkeitsdurchlässige Oberflächenlage (3), eine flüssigkeitsdichte Oberflächenlage (5) und einen ersten Absorptionskörper (7), der zwischen den Oberflächenlagen angeordnet ist und sich in einer Längsrichtung (11) und einer Querrichtung (9) entlang der Oberflächenlagen und auch in einer Durchgangsrichtung (10) von der flüssigkeitsdurchlässigen Oberflächenlage (3) in Richtung der flüssigkeitsdichten Oberflächenlage (5) erstreckt, wobei der erste Absorptionskörper (7) Öffnungen (29) aufweist, die sich in der Durchgangsrichtung (10) durch den ersten Absorptionskörper (7) erstrecken, und eine von Flüssigkeit durchdringbare Lage (31) auf der Seite des ersten Absorptionskörpers (7) angeordnet ist, die von der flüssigkeitsdurchlässigen Oberflächenlage (3) weg weist, **dadurch gekennzeichnet, dass** die flüssigkeitsdurchlässige Oberflächenlage (3) in den Öffnungen (29) durch die erste Absorptionslage (7) an der von Flüssigkeit durchdringbaren Lage (31) angebracht ist.

2. Absorbierender Gegenstand nach Anspruch 1, **dadurch gekennzeichnet, dass** die flüssigkeitsdurchlässige Oberflächenlage (3) nicht **dadurch** angebracht ist, dass sie absichtlich mit dem ersten Absorptionskörper (7) verbunden ist.

3. Absorbierender Gegenstand nach einem der vorstehenden Ansprüche, **dadurch gekennzeichnet, dass** der erste Absorptionskörper (7) nicht **dadurch** angebracht ist, dass er absichtlich mit der von Flüssigkeit durchdringbaren Lage (31) verbunden ist.

4. Absorbierender Gegenstand nach einem der vorstehenden Ansprüche, **dadurch gekennzeichnet, dass** die Öffnungen (29) in Form abgerundeter Löcher vorgesehen sind.

5. Absorbierender Gegenstand nach einem der vorstehenden Ansprüche, **dadurch gekennzeichnet, dass** die Öffnungen (29) in Form von Längskanälen vorgesehen sind.

6. Absorbierender Gegenstand nach einem der vorstehenden Ansprüche, **dadurch gekennzeichnet, dass** die flüssigkeitsdurchlässige Oberflächenlage (3) Löcher (32) aufweist, die an den Öffnungen (29) durch den ersten Absorptionskörper angeordnet sind, so dass Urin und/oder Durchfall durch die Löcher (32) und die Öffnungen (29) dringen kann.

7. Absorbierender Gegenstand nach einem der vorstehenden Ansprüche, **dadurch gekennzeichnet, dass** die flüssigkeitsdurchlässige Oberflächenlage (3) hydrophobe Bereiche zwischen den Öffnungen (29) durch den ersten Absorptionskörper (7) umfasst.

8. Absorbierender Gegenstand nach einem der vorstehenden Ansprüche, **dadurch gekennzeichnet, dass** die flüssigkeitsdurchlässige Oberflächenlage (3) ein maximales Flächengewicht von 20 g/m³ aufweist.

9. Absorbierender Gegenstand nach einem der vorstehenden Ansprüche, **dadurch gekennzeichnet, dass** eine Flüssigkeitstransportlage (40) zwischen der flüssigkeitsdurchlässigen Oberflächenlage (3) und dem ersten Absorptionskörper (7) angeordnet ist, wobei die Flüssigkeitstransportlage (40) auch durch die Öffnungen (29) an der von Flüssigkeit durchdringbaren Lage (31) angebracht ist.

10. Absorbierender Gegenstand nach einem der vorstehenden Ansprüche, **dadurch gekennzeichnet, dass** ein flexibler Raum (37) zwischen der von Flüssigkeit durchdringbaren Lage (31) und einer darunter liegenden Lage (5) ausgebildet ist.

## Revendications

1. Article absorbant, tel qu'une couche-culotte ou une protection contre l'incontinence, comprenant une couche de surface (3) perméable aux liquides, une couche de surface (5) imperméable aux liquides, et un premier corps absorbant (7) qui est agencé entre les couches de surface et qui s'étend dans une direction longitudinale (11) et dans une direction transversale (9) le long des couches de surface et également dans une direction d'épaisseur (10) depuis la couche de surface perméable aux liquides (3) en direction de la couche de surface imperméable aux liquides (5), ledit premier corps absorbant (7) comportant des ouvertures (29) qui s'étendent à travers le premier corps absorbant (7) dans la direction d'épaisseur (10) et une couche (31) pénétrable par les liquides étant disposée sur la face du premier corps absorbant (7) qui est orientée à l'écart de la couche de surface perméable aux liquides (3), **caractérisé en ce que** la couche de surface perméable aux liquides (3) est fixée à la couche (31) pénétrable par les liquides dans les ouvertures (29) passant à travers la première couche absorbante (7).

2. Article absorbant selon la revendication 1, **caractérisé en ce que** la couche de surface perméable aux liquides (3) n'est pas fixée, en étant intentionnellement assemblée avec le premier corps absorbant (7).

3. Article absorbant selon l'une quelconque des revendications qui précèdent, **caractérisé en ce que** le premier corps absorbant (7) n'est pas fixé en étant intentionnellement assemblé avec la couche (31) pénétrable par les liquides.

4. Article absorbant selon l'une quelconque des revendications qui précèdent, **caractérisé en ce que** les ouvertures (29) se présentent sous forme de trous arrondis.

5. Article absorbant selon l'une quelconque des revendications qui précèdent, **caractérisé en ce que** les ouvertures (29) se présentent sous forme de canaux longitudinaux.

6. Article absorbant selon l'une quelconque des revendications qui précèdent, **caractérisé en ce que** la couche de surface perméable aux liquides (3) comporte des trous (32) agencés dans les ouvertures (29) traversant le premier corps absorbant de telle manière que l'urine et/ou les selles peuvent passer dans les trous (32) et les ouvertures (29).

7. Article absorbant selon l'une quelconque des revendications qui précèdent, **caractérisé en ce que** la couche de surface perméable aux liquides (3) comprend des zones hydrophobes entre les ouvertures (29) qui traversent le premier corps absorbant (7).

8. Article absorbant selon l'une quelconque des revendications qui précèdent, **caractérisé en ce que** la couche de surface perméable aux liquides (3) a une masse surfacique maximum de 20 g/m².

9. Article absorbant selon l'une quelconque des revendications qui précèdent, **caractérisé en ce qu'**une couche (40) de transport de liquides est agencée entre la couche de surface perméable aux liquides (3) et le premier corps absorbant (7), la couche (40) de transport de liquides étant également fixée à la couche (31) pénétrable par les liquides dans les ouvertures (29).

10. Article absorbant selon l'une quelconque des revendications qui précèdent, **caractérisé en ce qu'**un espace (37) pouvant se déformer est formé entre la couche (31) pénétrable par les liquides et une couche sous-jacente (5).
